# FASCICULE DE BREVET EUROPEEN

(11) **EP 0 773 919 B1**
(45) Date de publication et mention de la délivrance du brevet: **20.09.2000**
(21) Numéro de dépôt: 96917543.9
(22) Date de dépôt: 24.05.1996
(51) Int. Cl.: C07C 65/30, C07C 51/255

(54) **PROCEDE DE PREPARATION DE 3-CARBOXY-4-HYDROXYBENZALDEHYDES ET DERIVES**
VERFAHREN ZUR HERSTELLUNG VON 3-CARBOXY-4-HYDROXY-BENZALDEHYDEN UND DERIVATEN
METHOD FOR PREPARING 3-CARBOXY-4-HYDROXYBENZALDEHYDES AND DERIVATIVES THEREOF

(30) Priorité: 24.05.1995 FR 9506186
(43) Date de publication de la demande: 21.05.1997
(73) Titulaire: RHODIA CHIMIE, 92408 Courbevoie Cédex (FR)
(72) Inventeur: METIVIER, Pascal, F-69110 Sainte-Foy-lès-Lyon (FR)
(74) Mandataire: Dutruc-Rosset, Marie-Claude
(86) Numéro de dépôt international: FR9600779
(87) Numéro de publication internationale: WO9637454

(56) Documents cités:
- EP-A- 0 028 714
- DE-C- 72 600
- FR-A- 2 011 316
- GB-A- 774 696

## Description

La présente invention a pour objet un procédé de préparation de 3-carboxy-4-hydroxybenzaldéhydes et dérivés, à partir de composés phénoliques porteurs de groupes formyle et/ou hydroxyméthyle en position 2 et 4.

Un autre objet de l'invention est la préparation de 4-hydroxybenzaldéhydes à partir de 3-carboxy-4-hydroxybenzaldéhydes.

L'invention concerne plus particulièrement la préparation du 3-méthoxy-4-hydroxybenzaldéhyde et du 3-éthoxy-4-hydroxybenzaldéhyde dénommés respectivement "vanilline" et "éthylvanilline".

La vanilline est obtenue principalement à partir de sources naturelles telles que la lignine mais une partie est préparée par voie chimique.

De nombreuses méthodes de préparation sont décrites dans la littérature [KIRK-OTHMER - Encyclopedia of Chemical Technology 23, p 1710, 3^{éme} édition] et plusieurs d'entre elles partent du gaïacol ou 2-méthoxyphénol.

Ainsi, on peut mentionner la préparation de la vanilline par réaction du gaïacol et de l'acide glyoxylique, oxydation à l'air du condensat, puis libération de la vanilline du milieu réactionnel par acidification. L'inconvénient dont souffre ce procédé est de faire appel à l'acide glyoxylique qui est un réactif coûteux.

Une autre voie d'accès à la vanilline selon la réaction de Reimer-Tiemann consiste à faire réagir le gaïacol et le chloroforme en présence d'hydroxyde de potassium. La formation de résine est un désavantage de cette méthode de préparation.

Selon la réaction de Gatterman, la vanilline est synthétisée par action de l'acide cyanhydrique sur le gaïacol, en présence d'acide chlorhydrique. Outre la mise en oeuvre d'un réactif de manipulation délicate, ce procédé présente l'inconvénient de ne pas être sélectif car la vanilline est accompagnée d'isovanilline et d'o-vanilline.

Une difficulté majeure présente dans la synthèse de la vanilline est de fixer un groupe formyle sur le gaïacol, sélectivement en position para du groupe hydroxyle.

Un autre problème à résoudre est de fournir un procédé compétitif d'un point de vue industrielle.

La présente invention propose un nouveau procédé permettant d'obvier aux inconvénients précités tout en satisfaisant aux exigences mentionnées ci-dessus.

Un premier objet de la présente invention est un procédé de préparation d'un 3-carboxy-4-hydroxybenzaldéhyde et dérivé caractérisé par le fait que l'on soumet un composé phénolique porteur de groupes formyle et/ou hydroxyméthyle en position 2 et 4 à une oxydation sélective du groupe en position 2 en groupe carboxylique, et éventuellement d'un groupe hydroxyméthyle en position 4 en groupe formyle, en présence d'une base et d'un catalyseur à base d'un métal M₁ choisi parmi les métaux du groupe 1b et 8.

Un autre objet de l'invention est la préparation d'un 4-hydroxybenzaldéhyde selon un procédé de décarboxylation du 3-carboxy-4-hydroxybenzaldéhyde.

Le procédé de l'invention convient tout à fait bien à la préparation de la vanilline. En effet, il permet d'effectuer l'oxydation du 4,6-diformylgaïacol ou du 4,6-di(hydroxyméthyl)gaïacol ou du 4-formyl-6-hydroxyméthylgaïacol ou du 4-hydroxyméthyl-6-formylgaïacol sélectivement en 5-carboxy-vanilline puis l'élimination du groupe carboxylique situé en position 5, conduit ainsi à la vanilline.

D'une manière similaire, il est appliqué au guétol (2-éthoxyphénol) conduisant alors à l'éthylvanilline.

Ce procédé n'est pas seulement sélectif mais aussi très compétitif d'un point de vue industriel car il fait appel à des réactifs peu onéreux.

Le substrat de départ intervenant dans le procédé de l'invention est un composé phénolique porteur de groupes formyle et/ou hydroxyméthyle en position 2 et 4.

Par "composé phénolique", on entend tout composé aromatique, dont le noyau aromatique est porteur d'un groupe hydroxyle.

Dans l'exposé qui suit de la présente invention, on entend par "aromatique", la notion classique d'aromaticité telle que définie dans la littérature, notamment par Jerry MARCH, Advanced Organic Chemistry, 4^{ème} édition, John Wiley and Sons, 1992, pp. 40 et suivantes.

Ce composé phénolique est porteur en position 2 et 4, de groupes hydroxyméthyle et/ou formyle.

Les composés phénoliques mis en oeuvre préférentiellement dans le nrocédé de l'invention répondent à la formule générale (II): 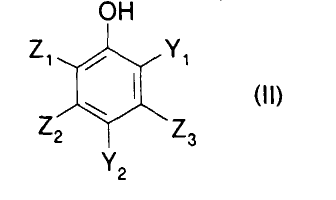 dans ladite formule (II):
- Y₁ et Y₂, identiques ou différents, représentent l'un des groupes suivants :
   . un groupe - CHO,
   . un groupe - CH₂OH,
- Z₁, Z₂ et Z₃, identiques ou différents, représentent un atome d'hydrogène, un radical alkyle, alcényle, alkoxy, hydroxyalkyle, alkoxyalkyle, cycloalkyle, aryle, un groupe hydroxyle, un groupe nitro, un atome d'halogène, un groupe trifluorométhyle.

Les composés qui conviennent particulièrement bien à la mise en oeuvre du procédé de l'invention répondent à la formule (II) dans laquelle Z₁, Z₂ et Z₃, identiques ou différents, représentent l'un des atomes ou groupes suivants :
. un atome d'hydrogène,
. un radical alkyle, linéaire ou ramifié, ayant de 1 à 12 atomes de carbone, de préférence de 1 à 4 atomes de carbone, tel que méthyle, éthyle, propyle, isopropyle, butyle, isobutyle, sec-butyle, tert-butyle,
. un radical alcényle, linéaire ou ramifié, ayant de 2 à 12 atomes de carbone, de préférence de 2 à 4 atomes de carbone, tel que vinyle, allyle,
. un radical alkoxy linéaire ou ramifié ayant de 1 à 12 atomes de carbone, de préférence de 1 à 4 atomes de carbone tel que les radicaux méthoxy, éthoxy, propoxy, isopropoxy, butoxy, isobutoxy, sec-butoxy, tert-butoxy,
. un radical phényle,
. un atome d'halogène, de préférence un atome de fluor, chlore ou brome.

La présente invention n'exclut pas la présence sur le cycle aromatique de substituants d'une nature différente dans la mesure où ils n'interfèrent pas avec les réactions du procédé de l'invention.

La présente invention s'applique préférentiellement aux composés de formule (II) dans laquelle Z₁ représente un radical alkoxy, linéaire ou ramifié, ayant de 1 à 6 atomes de carbone, de préférence, 1 à 4 atomes de carbone ; Z₂ et Z₃ représentant un atome d'hydrogène ; Y₁ et Y₂, identiques, représentent un groupe formyle ou un groupe hydroxyméthyle.

Comme exemples préférés de substrats mis en oeuvre dans le procédé de l'invention, on peut citer, entre autres :
- le 2,4-diformylphénol,
- le 1,2-dihydroxy-3,5-diformylbenzène,
- le 1-hydroxy-2-méthoxy-4,6-diformylbenzène [4,6-diformylgaïacol]
- le 1-hydroxy-2-éthoxy-4,6-diformylbenzène [4,6-diformylguétol]
- le 4,6-di(hydroxyméthyl)gaïacol,
- le 4,6-di(hydroxyméthyl)guétol.

Parmi les substrats précités, le 4,6-diformylgaïacol et le 4,6-di(hydroxyméthyl)gaïacol sont mis en oeuvre préférentiellement.

Conformément au procédé de l'invention, on part d'un composé phénolique qui est préférentiellement un composé répondant à la formule (II).

On donne ci-après, le schéma réactionnel du procédé de l'invention pour faciliter la compréhension de l'exposé de l'invention, sans pour autant lier la portée de l'invention, à celui-ci. 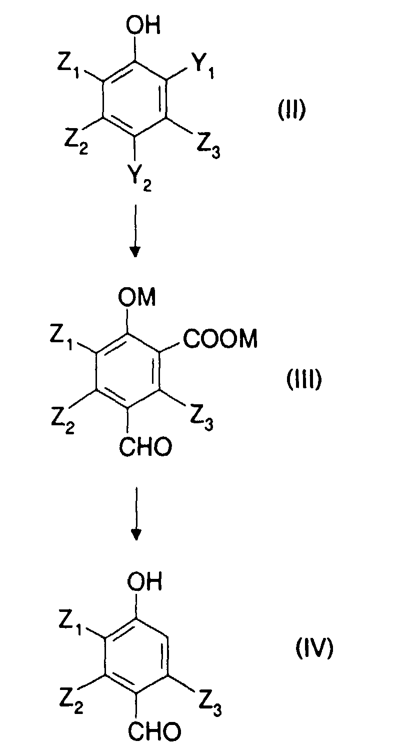 dans lesdites formules (II) à (IV) :
- M représente un atome d'hydrogène et/ou un cation métallique du groupe (Ia) ou (IIa) de la classification périodique ou un cation ammonium,
- Z₁, Z₂, Z₃, ayant les significations données précédemment.

Dans le présent texte, on se réfère ci-après à la Classification périodique des éléments publiée dans le Bulletin de la Société Chimique de France, n°1 (1966).

Conformément au procédé de l'invention, on effectue une oxydation sélective du groupe Y₁ en position 2 en groupe carboxylique d'un composé phénolique porteur de groupes formyle et/ou hydroxyméthyle en position 2 et 4, répondant préférentiellement à la formule (II) et éventuellement d'un groupe hydroxyméthyle en position 4 en groupe formyle.

L'oxydation est réalisée par l'oxygène moléculaire ou un gaz en contenant, en présence généralement d'un catalyseur.

Un mode d'oxydation préférée consiste à oxyder le composé phénolique de formule (II), en phase liquide, à l'aide d'oxygène moléculaire ou un gaz en contenant, en milieu aqueux renfermant un agent basique, en présence d'un catalyseur à base d'un métal M₁ choisi parmi les métaux du groupe 1b et 8 de la classification périodique des éléments comprenant éventuellement, à titre d'activateurs des métaux tels que le cadmium, le cérium, le bismuth, le plomb, l'argent, le tellure ou l'étain.

Conformément à l'invention, il a été trouvé d'une manière totalement surprenante que, si l'on augmentait la température et que l'on conduisait la réaction de préférence sous pression ou si l'on augmentait la quantité de la base présente lors de l'oxydation, on oxydait sélectivement les groupes formyle et/ou hydroxyméthyle en position 2 en groupe carboxylique, et le groupe situé en position 4 étant au plus oxydé en groupe aldéhyde.

Les catalyseurs intervenant dans le procédé de l'invention sont à base d'un métal du groupe 1b et 8 de la classification périodique.

Comme exemples de catalyseurs à base d'un métal du groupe 8 de la classification périodique, on peut citer le nickel, le ruthénium, le rhodium, le palladium, l'osmium, l'iridium, le platine et leurs mélanges. Pour ce qui est des métaux du groupe 1 b, on préfère faire appel au cuivre.

On utilise de préférence, des catalyseurs de platine et/ou de palladium, pris sous toutes les formes disponibles telles que par exemple : le noir de platine, le noir de palladium, l'oxyde de platine, l'oxyde de palladium ou le métal noble lui-même déposé sur des supports divers tels que le noir de carbone, le carbonate de calcium, les alumines et silices activées ou des matériaux équivalents. Des masses catalytiques à base de noir de carbone conviennent particulièrement.

La quantité de ce catalyseur à mettre en oeuvre, exprimée en poids de métal M₁ par rapport à celui du composé phénolique de formule (II) peut varier de 0,01 à 10 % et, de préférence, de 0,04 à 2 %.

Pour plus de détails sur les catalyseurs, on peut se référer à US-A-3 673 257, FR-A-2 305 420, FR-A-2 350 323.

L'activateur peut être choisi parmi tous ceux mentionnés dans les brevets précités. De préférence, on fait appel au bismuth, au plomb et au cadmium, sous forme de métaux libres ou de cations. Dans ce dernier cas, l'anion associé n'est pas critique et on peut utiliser tous dérivés de ces métaux. De préférence, on met en oeuvre le bismuth métal ou ses dérivés.

On peut faire appel à un dérivé minéral ou organique du bismuth dans lequel l'atome de bismuth se trouve à un degré d'oxydation supérieur à zéro, par exemple égal à 2, 3, 4 ou 5. Le reste associé au bismuth n'est pas critique dès l'instant qu'il satisfait à cette condition. L'activateur peut être soluble ou insoluble dans le milieu réactionnel.

Des composés illustratifs d'activateurs qui peuvent être utilisés dans le procédé selon la présente invention sont : les oxydes de bismuth ; les hydroxydes de bismuth ; les sels d'hydracides minéraux tels que : chlorure, bromure, iodure, sulfure, séléniure, tellure de bismuth ; les sels d'oxyacides minéraux tels que : sulfite, sulfate, nitrite, nitrate, phosphite, phosphate, pyrophosphate, carbonate, perchlorate, antimoniate, arséniate, sélénite, séléniate de bismuth ; les sels d'oxyacides dérivés de métaux de transition tels que : vanadate, niobate, tantalate, chromate, molybdate, tungstate, permanganate de bismuth.

D'autres composés appropriés sont également des sels d'acides organiques aliphatiques ou aromatiques tels que : acétate, propionate, benzoate, salicylate, oxalate, tartrate, lactate, citrate de bismuth ; des phénates tels que : gallate et pyrogallate de bismuth. Ces sels et phénates peuvent être aussi des sels de bismuthyle.

Comme autres composés minéraux ou organiques, on peut utiliser des combinaisons binaires du bismuth avec des éléments tels que phosphore et arsenic; des hétéropolyacides contenant du bismuth ainsi que leurs sels ; conviennent également les bismuthines aliphatiques et aromatiques.

A titre d'exemples spécifiques, on peut citer:
- comme oxydes : BiO ; Bi₂O₃ : Bi₂O₄ ; Bi₂O₅.
- comme hydroxydes : Bi(OH)₃,
- comme sels d'hydracides minéraux : le chlorure de bismuth BiCl₃ ; le bromure de bismuth BiBr₃ ; l'iodure de bismuth Bil₃ ; le sulfure de bismuth Bi₂S₃ ; le séléniure de bismuth Bi₂Se₃ : le tellure de bismuth Bi₂Te₃,
- comme sels d'oxyacides minéraux : le sulfite basique de bismuth Bi₂(SO₃)₃, Bi₂O₃, 5H₂O ; le sulfate neutre de bismuth Bi₂(SO₄)₃ ; le sulfate de bismuthyle (BiO)HSO₄ ; le nitrite de bismuthyle (BiO)NO₂, 0,5H₂O ; le nitrate neutre de bismuth Bi(NO₃)₃, 5H₂O ; le nitrate double de bismuth et de magnésium 2Bi(NO₃)₃, 3Mg(NO₃)₂, 24H₂O ; le nitrate de bismuthyle (BiO)NO₃ ; le phosphite de bismuth Bi₂(PO₃H)₃, 3H₂O ; le phosphate neutre de bismuth BiPO₄ ; le pyrophosphate de bismuth Bi₄(P₂O₇)₃ ; le carbonate de bismuthyle (BiO)₂CO₃, O,5H₂O ; le perchlorate neutre de bismuth Bi(ClO₄)₃, 5H₂O ; le perchlorate de bismuthyle (BiO)ClO₄ ; l'antimoniate de bismuth BiSbO₄ ; l'arséniate neutre de bismuth Bi(AsO₄)₃ ; l'arséniate de bismuthyle (BiO)AsO₄, 5H₂O ; le sélénite de bismuth Bi₂(SeO₃)₃.
- comme sels d'oxyacides dérivés de métaux de transtion : le vanadate de bismuth BiVO₄ ; le niobate de bismuth BiNbO₄ : le tantalate de bismuth BiTaO₄ ; le chromate neutre de bismuth Bi₂(CrO₄) ; le dichromate de bismuthyle [(BiO)₂]₂Cr₂O₇ ; le chromate acide de bismuthyle H(BiO)CrO₄ ; le chromate double de bismuthyle et de potassium K(BiO)CrO₄ ; le molybdate de bismuth Bi₂(MoO₄)₃ ; le tungstate de bismuth Bi₂(WO₄)₃ ; le molybdate double de bismuth et de sodium NaBi(MoO₄)₂ ; le permanganate basique de bismuth Bi₂O₂(OH)MnO₄.
- comme sels d'acides organiques aliphatiques ou aromatiques : l'acétate de bismuth Bi(C₂H₃O₂)₃ ; le propionate de bismuthyle (BiO)C₃H₅O₂ ; le benzoate basique de bismuth C₆H₅CO₂Bi(OH)₂ ; le salicylate de bismuthyle C₆H₄CO₂(BiO)(OH) ; l'oxalate de bismuth (C₂O₄)₃Bi₂ ; le tartrate de bismuth Bi₂(C₄H₄O₆)₃, 6H₂O ; le lactate de bismuth (C₆H₉O₅)OBi, 7H₂O ; le citrate de bismuth C₆H₅O₇Bi.
- comme phénates : le gallate basique de bismuth C₇H₇O₇Bi ; le pyrogallate basique de bismuth C₆H₃(OH)₂(OBi)(OH).

Comme autres composés minéraux ou organiques conviennent également : le phosphure de bismuth BiP ; l'arséniure de bismuth Bi₃As₄ ; le bismuthate de sodium NaBiO₃ ; les acides bismuth-thiocyaniques H₂[Bi(BNS)₅], H₃[Bi(CNS)₆] et leurs sels de sodium et potassium ; la triméthylbismuthine Bi(CH₃)₃, la triphénylbismuthine Bi(C₆H₅)₃.

Les dérivés du bismuth qui sont utilisés de préférence pour conduire le procédé selon l'invention sont : les oxydes de bismuth ; les hydroxydes de bismuth ; les sels de bismuth ou de bismuthyle d'hydracides minéraux ; les sels de bismuth ou de bismuthyle d'oxyacides minéraux ; les sels de bismuth ou de bismuthyle d'acides organiques aliphatiques ou aromatiques ; et les phénates de bismuth ou de bismuthyle.

Un groupe d'activateurs qui conviennent particulièrement bien à la réalisation de l'invention est constitué par : les oxydes de bismuth Bi₂O₃ et Bi₂O₄; l'hydroxyde de bismuth Bi(OH)₃; le sulfate neutre de bismuth Bi₂(SO₄)₃ ; le chlorure de bismuth BiCl₃ ; le bromure de bismuth BiBr₃ ; l'iodure de bismuth Bil₃ ; le nitrate neutre de bismuth Bi(NO₃)₃, 5H₂O ; le nitrate de bismuthyle BiO(NO₃) ; le carbonate de bismuthyle (BiO)₂CO₃, O,5H₂O ; l'acétate de bismuth Bi(C₂H₃O₂)₃ ; le salicylate de bismuthyle C₆H₄CO₂(BiO)(OH).

La quantité d'activateur utilisée, exprimée par la quantité de métal contenue dans l'activateur par rapport au poids du métal M₁ engagé, peut varier dans de larges limites. Par exemple, cette quantité peut être aussi petite que 0,1 % et peut atteindre le poids de métal M₁ engagé et même le dépasser sans inconvénient.

Plus particulièrement, cette quantité est choisie de manière qu'elle apporte dans le milieu d'oxydation de 10 à 900 ppm en poids de métal activateur par rapport au composé phénolique de formule (II). A cet égard, des quantités supérieures d'activateur de l'ordre de 900 à 1500 ppm peuvent naturellement être utilisées, mais sans avantage supplémentaire important.

Selon le procédé de l'invention, l'oxydation est conduite dans un milieu aqueux contenant en solution un agent basique, et plus particulièrement l'hydroxyde d'ammonium, les bases alcalines ou alcalino-terreuses parmi lesquelles on peut citer des hydroxydes tels que l'hydroxyde de sodium, de potassium, de lithium et la baryte ; des alcanolates alcalins tels que le méthylate, l'éthylate, l'isopropylate et le t-butylate de sodium ou de potassium, des carbonates ou bicarbonates de sodium ou de potassium et de façon générale, les sels des bases alcalines ou alcalino-terreuses et d'acides faibles.

Ainsi, les composés de formule (III) peuvent être totalement ou partiellement salifiés selon la quantité d'agent basique mise en oeuvre. Il s'ensuit que dans la formule (III) M symbolise un atome d'hydrogène et/ou un cation métallique du groupe (Ia) ou (IIa) ou un cation ammonium.

Pour des considérations économiques, on fait appel à l'hydroxyde de sodium ou de potassium. La proportion de base minérale à utiliser peut être comprise entre 0,5 et 10 moles, de préférence, entre 1 et 4 moles, et encore plus préférentiellement entre 2 et 4 moles de base minérale par mole de composé phénolique de formule (II).

La concentration pondérale du composé phénolique de formule (II) dans la phase liquide est habituellement comprise entre 1 % et 60 %, de préférence entre 2 % et 30 %.

Pratiquement une manière d'exécuter le procédé consiste à mettre en contact avec de l'oxygène moléculaire ou un gaz en contenant, par exemple l'air, la solution renfermant le composé phénolique de formule (II), l'agent basique, le catalyseur à base de métal M₁, éventuellement l'activateur, selon les proportions indiquées ci-dessus.

On peut opérer à pression atmosphérique, mais l'on préfère travailler sous pression entre 1 et 20 bar.

Le mélange est ensuite agité à la température désirée jusqu'à consommation d'une quantité d'oxygène correspondant à celle nécessaire pour transformer le(s) groupe(s) hydroxyméthyle et/ou formyle en groupe carboxylique et éventuellement le groupe hydroxyméthyle en groupe formyle.

La température réactionnelle à adopter varie selon la stabilité thermique des produits à préparer.

Conformément à l'invention, la température est choisie de préférence, dans une gamme de température allant de 30°C à 200°C, de préférence, entre 40°C et 160°C.

La température est à adapter par l'Homme du Métier en fonction des conditions réactionnelles (en particulier quantité de base, nature du métal M₁, pression et agitation). Il a été trouvé notamment que plus la température est basse, plus la quantité d'agent basique à mettre en oeuvre est choisie élevée.

On précisera, à titre d'exemples, les conditions préférées dans le cas des métaux préférés, platine et palladium. Pour le platine, la température étant choisie entre 100°C à 160 °C, la quantité de base à utiliser est avantageusement comprise entre 1 et 3 moles, par mole de composé phénolique de formule (II). Dans le cas du palladium, la température peut être choisie entre 30°C et 200°C, de préférence, entre 30°C et 100°C et pour ce dernier intervalle, la quantité de base est préférentiellement de 2 à 4 moles par mole dudit composé phénolique.

En fin de réaction qui dure de préférence, entre 30 minutes et 6 heures, on récupère le 3-carboxy-4-hydroxybenzaldéhyde qui peut être partiellement ou totalement salifié et répondant préférentiellement à la formule (III).

Puis, après refroidissement s'il y a lieu, on sépare la masse catalytique de la masse réactionnelle, par exemple par filtration.

Dans la dernière étape du procédé de l'invention, on effectue une réaction de décarboxylation.

Pour ce faire, on acidifie le milieu résultant par addition d'un acide protonique d'origine minérale, de préférence l'acide chlorhydrique ou l'acide sulfurique ou d'un acide organique tel que par exemple, l'acide triflurométhanesulfonique ou l'acide méthanesulfonique, jusqu'à obtention d'un pH inférieur ou égal à 3.

On chauffe le milieu réactionnel à une température variant par exemple entre 120°C et 350°C et, de préférence, de 150°C et 220°C.

Le procédé est conduit, de préférence, sous la pression autogène des réactifs.

En fin de réaction, on refroidit le milieu réactionnel entre 20°C et 80°C.

On obtient un milieu bi-phasique constitué d'une phase organique comprenant d'une part, le 4-hydroxybenzaldéhyde répondant préférentiellement à la formule (IV) et éventuellement le substrat de départ de formule (II) et d'autre part une phase aqueuse saline.

On sépare les phases organique et aqueuse et l'on récupère le 4-hydroxybenzaldéhyde à partir de la phase organique, selon les techniques classiques de séparation, de préférence, par distillation ou par extraction à l'aide d'un solvant approprié.

Conformément au procédé de l'invention, un 3-carboxy-4-hydroxybenzaldéhyde est obtenu par oxydation sélective d'un composé phénolique porteur de groupes formyle et/ou hydroxyméthyle en position 2 et 4.

Ainsi, les substrats de départ répondent plus particulièrement aux formules (IIa), (IIb), (IIc) et (IId) données ci-après :

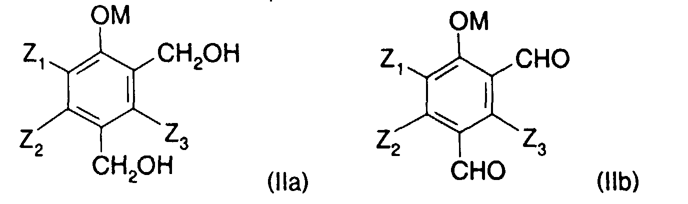

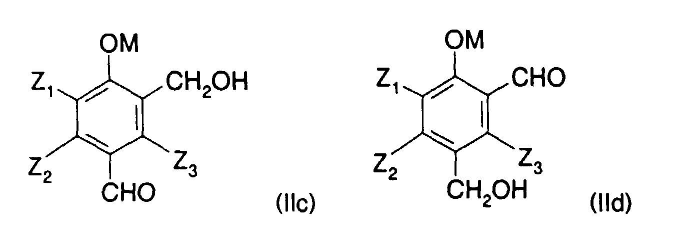

Lesdits composés, auxquels on peut appliquer le procédé selon l'invention sont des produits généralement connus qui peuvent être préparés par diverses méthodes de synthèse organique.

Ainsi, les composés de formule (IIb) peuvent-ils être préparés par oxydation de poly(hydroxyméthyl)phénols de formule (IIa) par l'oxygène moléculaire ou un gaz qui en contient, en phase aqueuse alcaline en présence d'un catalyseur à base d'un métal du groupe 8 de la classification périodique, de préférence, le platine et le palladium, contenant éventuellement à titre d'activateur des métaux tels que le cadmium, le cérium, le bismuth, le plomb, l'argent, le tellure ou l'étain. De tels procédés ont été décrits dans US-A-3 673 257, FR-A-2 305 420 et dans FR-A-2 350 323.

De leur côté, les poly(hydroxyméthyl)phénols sont des produits connus pour la plupart et qui peuvent être préparés par hydroxyméthylation de phénols substitués ou non au moyen du formaldéhyde ou de composés générateurs de formaldéhyde comme le paraformaldéhyde, dans les conditions les plus diverses : cf. notamment H.G. PEER Rec. Trav. Chim. Pays-Bas 79 825-835 (1960); GB-A-774696 ; GB-A-751845 ; EP-A-165 ; J.H. FREEMAN J. Am. Chem. Soc. 74 6 257-6 260 (1952) et 76 2080-2087 (1954) ; H.G. PEER Rec. Trav. Chim. Pays-Bas 78 851-863 (1959) ; H. EULER et al Arkiv für Chem. 13 1-7 (1939) ; P. CLAUS et al Monath. Chem. 103 1178-11293 (1972).

Un procédé d'hydroxyméthylation des phénols qui convient tout spécifiquement bien à la synthèse des poly(hydroxyméthyl)phénols utilisables pour la préparation des composés de formule (III) réside dans la condensation de formaldéhyde ou des composés générateurs de formaldéhyde avec un phénol en phase aqueuse en présence d'une base alcaline ou alcalino-terreuse.

Il apparaît particulièrement avantageux du point de vue industriel pour la mise en oeuvre du procédé selon la présente invention de faire appel à des composés de formule (IIb) obtenus par un procédé en deux étapes comprenant :
- l'hydroxyméthylation d'un phénol en milieu aqueux en présence d'une base alcaline ou alcalino-terreuse par le formaldéhyde ou un composé générateur de formaldéhyde, en un poly(hydroxyméthyl)phénol,
- et l'oxydation, sans séparation intermédiaire, des poly(hydroxyméthyl)phénols par l'oxygène moléculaire ou un gaz qui en contient, en phase aqueuse alcaline en présence d'un catalyseur à base d'un métal du groupe 8 de la classification périodique, éventuellement à titre d'activateur, un métal tel que ceux cités précédemment.

Plus spécifiquement encore le procédé selon la présente invention convient bien à la préparation de composés de formule (III) à partir de poly(hydroxyméthyl)phénols de formule (IIa) obtenus par un procédé en deux étapes comprenant :
- l'hydroxyméthylation d'un phénol non substitué sur les positions ortho et para par rapport au groupe hydroxyle, de formule générale (I): 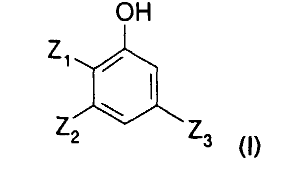 en poly(hydroxyméthyl)phénol comportant au moins un groupe hydroxyméthyle en position ortho et para par rapport au groupe hydroxyle, de formule générale (IIa) : 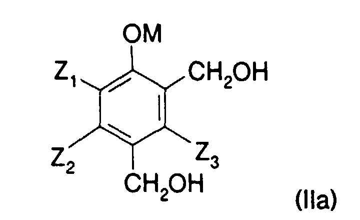 au moyen du formaldéhyde ou d'un générateur de formaldéhyde en phase aqueuse en présence d'une base alcaline ou alcalino-terreuse.
- l'oxydation en phase aqueuse alcaline des poly(hydroxyméthyl)phénols de formule (IIa) résultant de la première étape, par l'oxygène moléculaire ou un gaz qui en contient, en présence d'un catalyseur à base d'un métal du groupe 8 de la classification périodique, contenant éventuellement un métal tel que ceux utilisés à titre d'activateur, sans séparation intermédiaire des poly(hydroxyméthyl)phénols.

Parmi les phénols de formule (I) qui peuvent servir de point de départ à la synthèse des composés de formule (II), on peut citer le phénol, la pyrocatéchine, le gaïacol, le guétol, le 3-méthoxyphénol, le 3-éthoxyphénol, le 3-isopropoxyphénol, le 3-t-butyloxyphénol, le m-crésol, l'o-crésol.

Les conditions choisies pour le déroulement des étapes d'hydroxyméthylation et d'oxydation des poly(hydroxyméthyl)phénols sont celles enseignées par l'art antérieur rappelé ci-avant.

On peut faire appel au formaldéhyde ou tout générateur de formaldéhyde tel que, par exemple, le trioxane ou le paraformaldéhyde utilisé sous la forme de polyformaldéhydes linéaires de degré de polymérisation indifférent, ayant de préférence un nombre de motifs (CH₂O) compris entre 8 et 100 motifs,

Le formaldéhyde peut être utilisé, sous forme de solution aqueuse dont la concentration n'est pas critique. Elle peut varier entre 20 et 50 % en poids : on utilise de préférence les solutions commerciales dont la concentration est d'environ 30 à 40 % en poids.

La quantité de formaldéhyde exprimée en moles de formaldéhyde par mole de phénol peut varier dans de larges limites. Le rapport molaire formaldéhyde/phénol peut varier entre 1 et 8 et de préférence entre 2 et 4.

La quantité de base présente dans le milieu d'hydroxyméthylation exprimée par le nombre de moles de base/hydroxyle phénolique du phénol à hydroxyméthyler peut varier dans de larges limites. En général ce rapport, variable suivant la nature de la base, peut varier entre 0,1 et 2 et, de préférence, entre 0,5 et 1,1. Comme base, on peut utiliser celles citées plus haut pour la phase d'oxydation. L'emploi des hydroxydes alcalins en solution aqueuse est particulièrement commode.

En général, l'étape d'hydroxyméthylation est conduite à une température comprise entre 0 et 100°C et de préférence entre 20 et 70°C.

Le procédé est conduit, de préférence, sous la pression autogène des réactifs pour éviter les pertes éventuelles du paraformaldéhyde qui peut être gazeux aux températures de mises en oeuvre.

On préfère conduire la réaction sous atmosphère contrôlée de gaz inertes tels que l'azote ou les gaz rares, par exemple l'argon.

La durée de la réaction peut être très variable. Elle se situe, le plus souvent, entre 30 minutes et 24 heures, de préférence entre 4 heures et 8 heures.

D'un point de vue pratique, la réaction est aisément réalisée en chargeant dans l'appareillage le phénol et le formaldéhyde, éventuellement une base, puis en portant le mélange réactionnel sous agitation à la température désirée, pendant la durée nécessaire à l'achèvement de la réaction.

L'ordre d'introduction des réactifs n'est pas critique et peut donc être différent.

L'oxydation des poly(hydroxyméthyl)phénols en poly(formyl)phénols intermédiaires par l'oxygène moléculaire peut être conduite comme cela a été indiqué plus haut directement sur la solution aqueuse alcaline des sels des poly(hydroxyméthyl)phénols obtenus à l'étape d'hydroxyméthylation. Si cela est nécessaire, le pH de la solution est porté à une valeur comprise entre 8 et 13 par addition éventuelle d'une base alcaline ou alcalino-terreuse. La valeur optimale du pH dépend de la nature des poly(hydroxyméthyl)phénols.

La température de la réaction d'oxydation se situe entre 10°C et 100°C, et de préférence entre 20°C et 60°C.

A partir de composés phénoliques de formule (II), on obtient, selon l'invention un 3-carboxy-4-hydroxybenzaldéhyde qui, après décarboxylation conduit à un 4-hydroxybenzaldéhyde.

Comme mentionné précédemment, le procédé de l'invention est particulièrement bien adapté pour la préparation de la vanilline et de l'éthylvanilline.

On donne ci-après des exemples de réalisation de l'invention. Ces exemples sont donnés à titre illustratif et sans caractère limitatif.

Dans les exemples, on définit le taux de conversion et le rendement obtenu.

Le taux de conversion correspond au rapport entre le nombre de substrat transformées et le nombre de moles de substrat engagées.

Le rendement correspond au rapport entre le nombre de moles de produit formées et le nombre de moles de substrat engagées.

### EXEMPLES

### Exemple 1

Dans cet exemple, on effectue l'oxydation du 4,6-diformylgaïacol.

Dans un autoclave de 50 ml, muni d'une turbine d'agitation, on charge 0,502 g de 4,6-diformylgaïacol dosé à 86% (2,41 mmol), 3,3 ml d'une solution aqueuse d'hydroxyde de sodium à 33,4 g/l (2,75 mmol), 17 ml d'eau, 0,3 g de catalyseur comprenant du platine déposé sur charbon à raison de 2,5% en poids de métal, 20 mg de sulfate de bismuth [Bi₂(SO₄)₃].

On met sous pression d'air de 20 bar avec un débit de 3 l/h.

On chauffe le mélange réactionnel à 120°C pendant 2 heures.

On refroidit le mélange réactionnel et l'on ramène la pression à la pression atmosphérique, puis l'on filtre le catalyseur.

Le milieu réactionnel est alors dosé par chromatographie liquide haute performance.

Les résultats obtenus sont les suivants :
- la conversion du 4,6-diformylgaïacol est de 59 %,
- le rendement en 5-carboxyvanilline est de 59 %.

### Exemple 2

On réalise dans l'exemple ci-après, l'oxydation du 4-hydroxyméthyl-6-formylgaïacol.

Dans un autoclave de 50 ml muni d'une turbine d'agitation, on charge 0,507 g de 4-hydroxyméthyl-6-formylgaïacol (2,75 mmol), 1,1 ml d'une solution aqueuse d'hydroxyde de sodium à 7,69 mol/l (8,46 mmol), 19 ml d'eau, 0,3 g d'un catalyseur comprenant du platine déposé sur charbon à raison de 2,5% en poids de métal, 0,0185 g de sulfate de bismuth [Bi₂(SO₄)₃].

On met sous courant d'air (3 l/h), en maintenant la pression à 20 bar.

On chauffe 7 heures à 45°C, puis 5 heures 30 à 120°C.

On refroidit le mélange réactionnel et l'on ramène la pression à la pression atmosphérique, puis l'on filtre le catalyseur.

Le milieu réactionnel est alors dosé par chromatographie liquide haute performance.

Les résultats obtenus sont les suivants :
- la conversion du 4-hydroxyméthyl-6-formylgaïacol est totale,
- le rendement en 5-carboxyvanilline est de 52%,
- le rendement en acide 5-carboxyvanillique est de 4,5%,
- le rendement en 4,6-diformylgaïacol est de 29%.

### Exemple 3

Dans cet exemple, on effectue l'oxydation du 4-hydroxyméthyl-6-formylgaïacol.

Dans un autoclave de 50 ml muni d'une turbine d'agitation, on charge 0,507 g de 4-hydroxyméthyl-6-formylgaïacol (2,75 mmol), 1,1 ml d'une solution aqueuse d'hydroxyde de sodium à 7,69 mol/I (8,46 mmol), 19 ml d'eau, 0,3 g de catalyseur comprenant du platine déposé sur charbon à raison de 2,5% en poids de métal.

On met sous courant d'air (3 l/h), en maintenant la pression à 20 bar.

On chauffe 4 heures 30, à 120°C.

On refroidit le mélange réactionnel et l'on ramène la pression à la pression atmosphérique, puis l'on filtre le catalyseur.

Le milieu réactionnel est alors dosé par chromatographie liquide haute performance.

Les résultats obtenus sont les suivants :
- la conversion du 4-hydroxyméthyl-6-formylgaïacol est de 98%,
- le rendement en 5-carboxyvanilline est de 25%,
- le rendement en 4,6-diformylgaïacol est de 59%.

### Exemple 4

On réalise dans l'exemple suivant, l'oxydation du 4,6-di(hydroxyméthyl)gaïacol.

Dans un autoclave de 50 ml muni d'une turbine d'agitation, on charge 0,8832 g de 4,6-di(hydroxyméthyl)gaïacol à 75% (3,60 mmol), 1,1 ml d'une solution aqueuse d'hydroxyde de sodium à 7,69 mol/l (8,46 mmol), 19 ml d'eau, 0,44 g de catalyseur comprenant du palladium déposé sur charbon à raison de 3 % en poids de métal.

On met sous courant d'air (3 l/h), en maintenant la pression à 20 bar.

On chauffe 3 heures 40, à 120°C.

On refroidit le mélange réactionnel et l'on ramène la pression à la pression atmosphérique, puis l'on filtre le catalyseur.

Le milieu réactionnel est alors dosé par chromatographie liquide haute performance.

Les résultats obtenus sont les suivants :
- la conversion du 4,6-di(hydroxyméthyl)gaïacol est totale,
- le rendement en 5-carboxyvanilline est de 32%,
- le rendement en 4,6-diformylgaïacol est de 14%,
- le rendement en acide 5-carboxyvanillique est de 12%.

### Exemple 5

Dans cet exemple, on effectue l'oxydation du 4,6-di(hydroxyméthyl)gaïacol.

Dans un autoclave de 50 ml muni d'une turbine d'agitation, on charge 0,8947g de 4,6-di(hydroxyméthyl)gaïacol à 75% (3,64 mmol), 1,1 ml d'une solution aqueuse d'hydroxyde de sodium à 7,69 mol/l (8,46 mmol), 19 ml d'eau, 0,46 g de catalyseur comprenant du platine déposé sur charbon à raison de 2,5% en poids de métal.

On met sous courant d'air (3 l/h), en maintenant la pression à 20 bar.

On chauffe 3 heures 30, à 120°C.

On refroidit le mélange réactionnel et l'on ramène la pression à la pression atmosphérique, puis l'on filtre le catalyseur.

Le milieu réactionnel est alors dosé par chromatographie liquide haute performance.

Les résultats obtenus sont les suivants :
- la conversion du 4,6-dihydroxyméthylgaïacol est totale,
- le rendement en 5-carboxyvanilline est de 41%,
- le rendement en 4,6-diformylgaïacol est de 31%,
- le rendement en acide 5-carboxyvanillique est de 4,5%.

### Exemple 6

On exemplifie ci-après, la préparation de la vanilline selon un enchaînement comprenant l'oxydation du 4,6-di(hydroxyméthyl)gaïacol suivie d'une décarboxylation.

Dans un autoclave de 50 ml muni d'une turbine d'agitation, on charge 0,8952 g de 4,6-di(hydroxyméthyl)gaïacol à 75% (3,64 mmol), 1,1 ml d'une solution aqueuse d'hydroxyde de sodium à 7,69 mol/l (8,46 mmol), 19 ml d'eau, 0,44 g de catalyseur comprenant du platine déposé sur charbon à raison de 2,5% en poids de métal.

On met sous courant d'air (3 l/h), en maintenant la pression à 20 bar.

On chauffe 3 heures 40, à 120°C.

On refroidit le mélange réactionnel et l'on ramène la pression à la pression atmosphérique, puis l'on filtre le catalyseur.

Le milieu réactionnel est homogène.

On acidifie alors le milieu réactionnel par 10 ml d'une solution aqueuse d'acide sulfurique 2 N.

On chauffe le mélange réactionnel sous pression autogène, 25 min à 200°C.

On refroidit le milieu réactionnel, on dilue par de l'acétonitrile et on dose par chromatographie liquide haute performance.

Le rendement en vanilline obtenu est de 41 %.

### Exemple 7

Dans cet exemple, on effectue l'oxydation du 4,6-diformylgaïacol.

Dans un autoclave de 2000 ml, muni d'une turbine d'agitation, on charge 120 g de 4,6-diformylgaïacol (0,67 mol), 270 g d'une solution aqueuse d'hydroxyde de sodium à 30 % en poids (2 mol), 1600 ml d'eau, 21 g de catalyseur comprenant du palladium déposé sur charbon à raison de 3 % en poids de métal, 1,4 g de sulfate de bismuth [Bi₂(SO₄)₃].

On met sous pression d'air de 15 bar avec un débit de 600 l/h.

On chauffe le mélange réactionnel à 100°C pendant 1 heure.

On refroidit le mélange réactionnel et l'on ramène la pression à la pression atmosphérique, puis l'on filtre le catalyseur.

Le milieu réactionnel est alors dosé par chromatographie liquide haute performance.

Les résultats obtenus sont les suivants :
- la conversion du 4,6-diformylgaïacol est de 84 %,
- le rendement en 5-carboxyvanilline est de 82 %.

### Exemple 8

On effectue également dans cet exemple, l'oxydation du 4,6-diformylgaïacol.

Dans un autoclave de 2000 ml, muni d'une turbine d'agitation, on charge 120 g de 4,6-diformylgaïacol (0,67 mol), 210 g d'une solution aqueuse d'hydroxyde de sodium à 30 % en poids (2 mol), 1650 ml d'eau, 20 g de catalyseur comprenant du platine déposé sur charbon à raison de 3 % en poids de métal, 0,3 g de sulfate de bismuth [Bi₂(SO₄)₃].

On met sous pression d'air de 15 bar avec un débit de 600 l/h.

On chauffe le mélange réactionnel à 140°C pendant 3 heures.

On refroidit le mélange réactionnel et l'on ramène la pression à la pression atmosphérique, puis l'on filtre le catalyseur.

Le milieu réactionnel est alors dosé par chromatographie liquide haute performance.

Les résultats obtenus sont les suivants :
- la conversion du 4,6-diformylgaïacol est de 89 %,
- le rendement en 5-carboxyvanilline est de 81 %.

## Revendications

1. Procédé de préparation d'un 3-carboxy-4-hydroxybenzaldéhyde et dérivé caractérisé par le fait que l'on soumet un composé phénolique porteur de groupes formyle et/ou hydroxyméthyle en position 2 et 4 à une oxydation sélective du groupe en position 2 en groupe carboxylique, et éventuellement d'un groupe hydroxyméthyle en position 4 en groupe formyle, en présence d'une base et d'un catalyseur à base d'un métal M₁ choisi parmi les métaux du groupe 1b et 8.

2. Procédé selon la revendication 1 caractérisé par le fait que le composé phénolique mis en oeuvre répond à la formule générale (II): 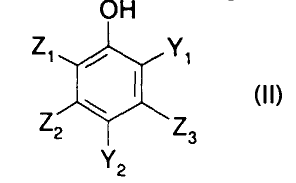 dans ladite formule (II):
- Y₁ et Y₂, identiques ou différents, représentent l'un des groupes suivants :
. un groupe - CHO,
. un groupe - CH₂OH,
- Z₁, Z₂ et Z₃, identiques ou différents, représentent un atome d'hydrogène, un radical alkyle, alcényle, alkoxy, hydroxyalkyle, alkoxyalkyle, cycloalkyle, aryle, un groupe hydroxyle, un groupe nitro, un atome d'halogène, un groupe trifluorométhyle.

3. Procédé selon l'une des revendications 1 et 2 caractérisé par le fait que le composé phénolique répond à la formule (II) dans laquelle Z₁, Z₂ et Z₃, identiques ou différents, représentent l'un des atomes ou groupes suivants :
. un atome d'hydrogène,
. un radical alkyle, linéaire ou ramifié, ayant de 1 à 12 atomes de carbone, de préférence de 1 à 4 atomes de carbone, tel que méthyle, éthyle, propyle, isopropyle, butyle, isobutyle, sec-butyle, tert-butyle,
. un radical alcényle, linéaire ou ramifié, ayant de 2 à 12 atomes de carbone, de préférence de 2 à 4 atomes de carbone, tel que vinyle, allyle,
. un radical alkoxy linéaire ou ramifié ayant de 1 à 12 atomes de carbone, de préférence de 1 à 4 atomes de carbone tel que les radicaux méthoxy, éthoxy, propoxy, isopropoxy, butoxy, isobutoxy, sec-butoxy, tert-butoxy,
. un radical phényle,
. un atome d'halogène, de préférence un atome de fluor, chlore ou brome.

4. Procédé selon l'une des revendications 1 à 3 caractérisé par le fait que le composé phénolique répond à la formule (II) dans laquelle Z₁ représente un radical alkoxy, linéaire ou ramifié, ayant de 1 à 6 atomes de carbone, de préférence, 1 à 4 atomes de carbone ; Z₂ et Z₃ représentant un atome d'hydrogène ; Y₁ et Y₂, identiques, représentent un groupe formyle ou un groupe hydroxyméthyle.

5. Procédé selon l'une des revendications 1 à 4 caractérisé par le fait que le composé phénolique répondant à la formule (II) est :
- le 2,4-diformylphénol,
- le 1,2-dihydroxy-3,5-diformylbenzène,
- le 1-hydroxy-2-méthoxy-4,6-diformylbenzène [4,6-diformylgaïacol]
- le 1-hydroxy-2-éthoxy-4,6-diformylbenzène [4,6-diformylguétol]
- le 4,6-di(hydroxyméthyl)gaïacol,
- le 4,6-di(hydroxyméthyl)guétol.

6. Procédé selon l'une des revendications 1 à 5 caractérisé par le fait que l'on oxyde le composé phénolique de formule (II), en phase liquide, à l'aide d'oxygène moléculaire ou un gaz en contenant, en milieu aqueux renfermant un agent basique, en présence d'un catalyseur à base d'un métal M₁ choisi parmi les métaux du groupe 1 b et 8 de la classification périodique des éléments, comprenant éventuellement, à titre d'activateurs des métaux tels que le cadmium, le cérium, le bismuth, le plomb, l'argent, le tellure ou l'étain.

7. Procédé selon la revendication 6 caractérisé par le fait que le catalyseur est à base de cuivre, de nickel, de ruthénium, de rhodium, de palladium, d'osmium, d'iridium, de platine ou de leurs mélanges : ledit catalyseur étant de préférence à base de platine et/ou de palladium.

8. Procédé selon l'une des revendications 6 et 7 caractérisé par le fait que le catalyseur au platine et/ou palladium, est apporté sous forme de noir de platine, de noir de palladium, d'oxyde de platine, d'oxyde de palladium ou de métal noble lui-même déposé sur des supports divers tels que le noir de carbone, le carbonate de calcium, les alumines et silices activées ou des matériaux équivalents, de préférence le noir de carbone.

9. Procédé selon l'une des revendications 6 à 8 caractérisé par le fait que la quantité de catalyseur à mettre en oeuvre, exprimée en poids de métal M₁ par rapport à celui du composé phénolique de formule (II) peut varier de 0,01 à 10 % et, de préférence, de 0,04 à 2 %.

10. Procédé selon l'une des revendication 6 à 9 caractérisé par le fait que l'activateur est un dérivé organique ou inorganique du bismuth pris dans le groupe formé par : les oxydes de bismuth ; les hydroxydes de bismuth ; les sels de bismuth ou de bismuthyle d'hydracides minéraux, de préférence les chlorure, bromure, iodure, sulfure, sélénure, tellure ; les sels de bismuth ou de bismuthyle d'oxyacides minéraux, de préférence les sulfite, sulfate, nitrite, nitrate, phosphite, phosphate, pyrophosphate, carbonate, perchlorate, antimoniate, arséniate, sélénite, séléniate ; les sels de bismuth ou de bismuthyle d'acides organiques aliphatiques ou aromatiques, de préférence les acétate, propionate, salicylate, benzoate, oxalate, tartrate, lactate, citrate ; les phénates de bismuth ou de bismuthyle, de préférence les gallate et pyrogallate.

11. Procédé selon la revendication 10 caractérisé par le fait que le dérivé du bismuth est pris dans le groupe formé par : les oxydes de bismuth Bi₂O₃ et Bi₂O₄ ; l'hydroxyde de bismuth Bi(OH)₃ ; le chlorure de bismuth BiCl₃ ; le bromure de bismuth BiBr₃ ; l'iodure de bismuth Bil₃ ; le sulfate neutre de bismuth Bi₂(SO₄)₃ ; le nitrate neutre de bismuth Bi(NO₃)₃, 5H₂O ; le nitrate de bismuthyle BiO(NO₃) ; le carbonate de bismuthyle (BiO)₂CO₃, 0,5 H₂O ; l'acétate de bismuth Bi(C₂H₃O₂)₃ ; le salicylate de bismuthyle C₆H₄CO₂(BiO)OH.

12. Procédé selon l'une des revendications 6 à 11 caractérisé par le fait que la quantité d'activateur est choisie de manière qu'elle apporte dans la milieu : d'une part, au moins 0,1 % en poids de métal activateur par rapport au poids du métal M₁ engagé, et d'autre part de 10 à 900 ppm en poids de métal M₁ par rapport au composé phénolique de formule (II)

13. Procédé selon l'une des revendications 6 à 12 caractérisé par le fait que la réaction d'oxydation est conduite dans une gamme de température allant de 30°C à 200°C, de préférence, entre 40°C et 160°C.

14. Procédé selon l'une des revendications 6 à 13 caractérisé par le fait que l'on opère sous pression entre 1 et 20 bar.

15. Procédé selon l'une des revendications 1 à 14 caractérisé par le fait que l'oxydation est conduite dans un milieu aqueux contenant en solution un agent basique, de préférence, un hydroxyde de sodium, de potassium, en une quantité telle qu'elle représente de 0,5 à 10 moles de base minérale par mole de composé phénolique de formule (II).

16. Procédé selon l'une des revendications 13 et 15 caractérisé par le fait que dans le cas d'un catalyseur au platine, la température est choisie entre 100°C et 160°C ; la quantité de base à utiliser étant comprise entre 1 et 3 moles, par mole de composés phénoliques de formule (II).

17. Procédé selon l'une des revendications 13 et 15 caractérisé par le fait que dans le cas d'un catalyseur au palladium, la température est choisie entre 30°C et 200°C, de préférence, entre 30°C et 100°C ; la quantité de base à utiliser étant comprise entre 2 et 4 moles, par mole de composés phénoliques de formule (II).

18. Procédé selon l'une des revendications 1 à 17 caractérisé par le fait que l'on effectue la décarboxylation d'un 3-carboxy-4-hydroxybenzaldéhyde qui peut être partiellement ou totalement salifié.

19. Procédé selon la revendication 18 caractérisé par le fait que le 3-carboxy-4-hydroxybenzaldéhyde répond à la formule générale (III): 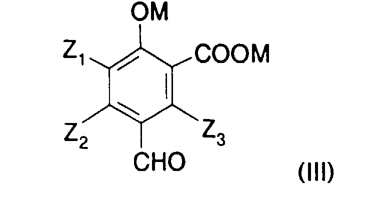 dans ladite formule (III):
- M représente un atome d'hydrogène et/ou un cation métallique du groupe (Ia) ou (IIa) ou un cation ammonium,
- Z₁, Z₂, Z₃, ayant les significations données dans les revendications 2 à 4.

20. Procédé selon l'une des revendications 18 et 19 caractérisé par le fait que l'on effectue la décarboxylation dudit acide par addition d'un acide protonique d'origine minérale, de préférence l'acide chlorhydrique ou l'acide sulfurique ou organique, jusqu'à obtention d'un pH inférieur ou égal à 3.

21. Procédé selon l'une des revendications 18 à 20 caractérisé par le fait que l'on chauffe le milieu réactionnel à une température variant entre 120°C et 350°C et, de préférence, de 150°C et 220°C et après refroidissement que l'on sépare le 4-hydroxybenzaldéhyde, et répondant préférentiellement à la formule (IV) : 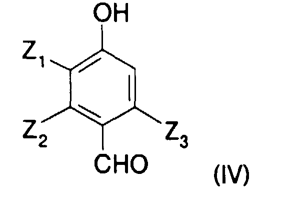 dans ladite formule (IV) :
- Z₁, Z₂, Z₃, ayant les significations données dans les revendications 2 à 4.

22. Procédé selon l'une des revendications 1 à 21 caractérisé par le fait que les substrats de départ répondent aux formules (IIa), (IIb) (llc) et (IId) ; 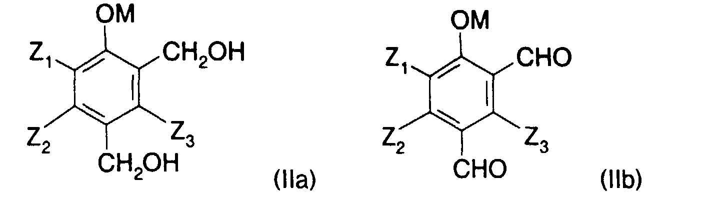 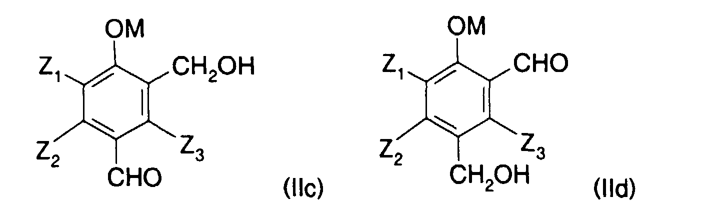 dans lesdites formules (IIa) à (IId) ;
- M représente un atome d'hydrogène et/ou un cation métallique du groupe (Ia) ou (IIa) ou un cation ammonium,
- Z₁, Z₂, Z₃, ayant les significations données dans les revendications 2 à 4.

23. Procédé selon la revendication 22 caractérisé par le fait que le composé de formule (IIb) est préparé par oxydation de poly(hydroxyméthyl)phénols de formule (IIa) par l'oxygène moléculaire ou un gaz qui en contient, en phase aqueuse alcaline en présence d'un catalyseur à base d'un métal du groupe 8 de la classification périodique, de préférence, le platine et le palladium, contenant éventuellement à titre d'activateur des métaux tels que le cadmium, le cérium, le bismuth, le plomb, l'argent, le tellure ou l'étain.

24. Procédé selon l'une des revendications 22 et 23 caractérisé par le fait que les poly(hydroxyméthyl)phénols de formule (IIa) sont obtenus par un procédé en deux étapes comprenant :
- l'hydroxyméthylation d'un phénol non substitué sur les positions ortho et para par rapport au groupe hydroxyle, de formule générale (I): 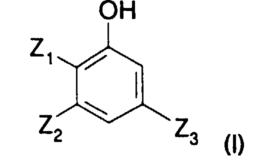 en poly(hydroxyméthyl)phénol comportant au moins un groupe hydroxyméthyle en position ortho et para par rapport au groupe hydroxyle, de formule générale (IIa) : 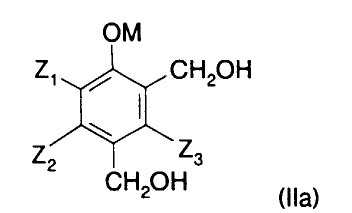 au moyen du formaldéhyde ou d'un générateur de formaldéhyde en phase aqueuse en présence d'une base alcaline ou alcalino-terreuse.
- l'oxydation en phase aqueuse alcaline des poly(hydroxyméthyl)phénols de formule (IIa) résultant de la première étape, par l'oxygène moléculaire ou un gaz qui en contient, en présence d'un catalyseur à base d'un métal du groupe 8 de la classification périodique, contenant éventuellement un métal tel que ceux utilisés à titre d'activateur, sans séparation intermédiaire des poly(hydroxyméthyl)phénols.

25. Procédé selon la revendication 24 caractérisé par le fait que le phénol de formule (I) est le phénol, la pyrocatéchine, le gaïacol, le guétol, le 3-méthoxyphénol, le 3-éthoxyphénol, le 3-isopropoxyphénol, le 3-t-butyloxyphénol, le m-crésol, l'o-crésol.

26. Procédé selon la revendication 24 caractérisé par le fait que la température de la réaction d'oxydation des composés de formule (IIa) se situe entre 10°C et 100°C, et de préférence entre 20°C et 60°C.

27. Procédé de préparation de la vanilline selon l'une des revendications 1 à 26 caractérisé par le fait que l'on oxyde du 4,6-diformylgaïacol ou du 4,6-di(hydroxyméthyl)gaïacol ou du 4-formyl-6-hydroxyméthylgaïacol ou du 4-hydroxyméthyl-6-formylgaïacol en 5-carboxy-vanilline, puis l'on élimine le groupe carboxylique situé en position 5, conduisant ainsi à la vanilline.

28. Procédé de préparation de l'éthylvanilline selon l'une des revendications 1 à 26 caractérisé par le fait que l'on oxyde du 4,6-diformylguétol ou du 4,6-di(hydroxyméthyl)guétol ou du 4-formyl-6-hydroxyméthylguétol ou du 4-hydroxyméthyl-6-formylguétol en 5-carboxy-éthylvanilline, puis l'on élimine le groupe carboxylique situé en position 5, conduisant ainsi à l'éthylvanilline.

## Patentansprüche

1. Verfahren zur Herstellung eines 3-Carboxy-4-hydroxybenzaldehyds und seiner Derivate, dadurch gekennzeichnet, daß man eine phenolische Verbindung, die ein Träger von Formyl- und/oder Hydroxymethylgruppen in 2- und 4-Stellung ist, einer selektiven Oxidation der Gruppe in 2-Stellung zur Carboxylgruppe und gegebenenfalls einer Hydroxymethylgruppe in 4-Stellung zur Formylgruppe in Gegenwart einer Base und eines Katalysators auf Grundlage eines Metalls M₁ unterzieht, das aus den Metallen der Gruppen 1b und 8 gewählt ist.

2. Verfahren nach Anspruch 1, dadurch gekennzeichnet, daß die eingesetzte phenolische Verbindung der allgemeinen Formel (II) entspricht: 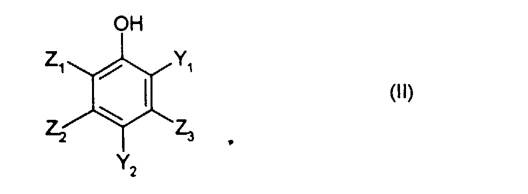 wobei in der genannten Formel (II):
- die gleichen oder verschiedenen Y₁ und Y₂ eine der folgenden Gruppen bedeuten:
• eine CHO-Gruppe,
• eine CH₂OH-Gruppe,
- die gleichen oder verschiedenen Z₁, Z₂ und Z₃ ein Wasserstoffatom, einen Alkyl-, Alkenyl-, Alkoxy-, Hydroxyalkyl-, Alkoxyalkyl-, Cycloalkyl-, Arylrest, eine Hydroxylgruppe, eine Nitrogruppe, ein Halogenatom, eine Trifluormethylgruppe bedeuten.

3. Verfahren nach Anspruch 1 oder 2, dadurch gekennzeichnet, daß die phenolische Verbindung der Formel (II) entspricht, in der die gleichen oder verschiedenen Z₁, Z₂ und Z₃ eine der folgenden Atome oder Gruppen bedeuten:
- ein Wasserstoffatom,
- einen linearen oder verzweigten Alkylrest mit 1 bis 12 Kohlenstoffatomen, vorzugsweise 1 bis 4 Kohlenstoffatomen, wie Methyl, Ethyl, Propyl, Isopropyl, Butyl, Isobutyl, sec.-Butyl, tert.-Butyl,
- einen linearen oder verzweigten Alkenylrest mit 2 bis 12 Kohlenstoffatomen, vorzugsweise 2 bis 4 Kohlenstoffatomen, wie Vinyl, Allyl,
- einen linearen oder verzweigten Alkoxyrest mit 1 bis 12 Kohlenstoffatomen, vorzugsweise 1 bis 4 Kohlenstoffatomen, wie die Methoxy-, Ethoxy-, Propoxy-, Isopropoxy-, Butoxy-, Isobutoxy-, sec.-Butoxy-, tert.-Butoxyreste,
- einen Phenylrest,
- ein Halogenatom, vorzugsweise ein Fluor-, Chlor- oder Bromatom.

4. Verfahren nach einem der Ansprüche 1 bis 3, dadurch gekennzeichnet, daß die phenolische Verbindung der Formel (II) entspricht, wobei in dieser Z₁ einen linearen oder verzweigten Alkoxyrest mit 1 bis 6 Kohlenstoffatomen, vorzugsweise 1 bis 4 Kohlenstoffatomen, bedeutet; Z₂ und Z₃ ein Wasserstoffatom bedeuten; die gleichen Y₁ und Y₂ eine Formyl- oder eine Hydroxymethylgruppe bedeuten.

5. Verfahren nach einem der Ansprüche 1 bis 4, dadurch gekennzeichnet, daß die der Formel (II) entsprechende phenolische Verbindung ist:
- 2,4-Diformylphenol,
- 1,2-Dihydroxy-3,5-diformylbenzol,
- 1-Hydroxy-2-methoxy-4,6-diformylbenzol[4,6-diformylguajakol],
- 1-Hydroxy-2-ethoxy-4,6-diformylbenzol[4,6-diformyl-2-ethoxyphenol],
- 4,6-Di(hydroxymethyl)guajakol,
- 4,6-Di(hydroxymethyl)-2-ethoxyphenol.

6. Verfahren nach einem der Ansprüche 1 bis 5, dadurch gekennzeichnet, daß man die phenolische Verbindung der Formel (II) in flüssiger Phase mit Hilfe von molekularem Sauerstoff oder einem diesen enthaltenden Gas in einem ein basisches Mittel enthaltenden wäßrigen Medium in Gegenwart eines Katalysators auf Grundlage eines Metalls M₁ oxidiert, das aus den Metallen der Gruppe 1b und 8 des Periodensystems gewählt ist und das gegebenenfalls als Aktivator Metalle wie Cadmium, Cerium, Bismut, Blei, Silber, Tellur oder Zinn enthält.

7. Verfahren nach Anspruch 6, dadurch gekennzeichnet, daß der Katalysator auf Basis von Kupfer, Nickel, Ruthenium, Rhodium, Palladium, Osmium, Iridium, Platin oder deren Gemischen ist: wobei der genannte Katalysator vorzugsweise auf Basis von Platin und/oder Palladium ist.

8. Verfahren nach Anspruch 6 oder 7, dadurch gekennzeichnet, daß der Platin-und/oder Palladiumkatalysator in Form von Platinschwarz, Palladiumschwarz, Platinoxid, Palladiumoxid oder dem Edelmetall selbst eingebracht wird, das auf verschiedenen Trägern wie Ruß, Calciumcarbonat, den aktivierten Aluminiumoxiden und Kieselsäuren oder äquivalenten Materialien, vorzugsweise Ruß, aufgebracht ist.

9. Verfahren nach einem der Ansprüche 6 bis 8, dadurch gekennzeichnet, daß die Menge des einzusetzenden Katalysators, definiert als Gewicht des Metalls M₁ im Verhältnis zu dem der phenolischen Verbindung der Formel (II), von 0,01 bis 10 %, vorzugsweise von 0,04 bis 2 %, reichen kann.

10. Verfahren nach einem der Ansprüche 6 bis 9, dadurch gekennzeichnet, daß der Aktivator ein organisches oder anorganisches Bismutderivat der Gruppe ist, die sich bildet aus: den Bismutoxiden; den Bismuthydroxiden; den Bismut- oder Bismutoxidsalzen der mineralischen Wasserstoffsäuren, vorzugsweise den Chloriden, Bromiden, lodiden, Sulfiden, Seleniden, Telluriden; den Bismut- oder Bismutoxidsalzen der mineralischen Oxosäuren, vorzugsweise der Sulfite, Sulfate, Nitrite, Nitrate, Phosphite, Phosphate, Pyrophosphate, Carbonate, Perchlorate, Antimonate, Arsenate, Selenite, Selenate; den Bismut- oder Bismutoxidsalzen der aliphatischen oder aromatischen organischen Säuren, vorzugsweise den Acetaten, Propionaten, Salicylaten, Benzoaten, Oxalaten, Tartraten, Lactaten, Citraten; den Bismut- oder Bismutoxidphenolaten, vorzugsweise den Gallussäureestern und Pyrogallolestern.

11. Verfahren nach Anspruch 10, dadurch gekennzeichnet, daß das Bismutderivat zur Gruppe gehört, die sich bildet aus: den Bismutoxiden Bi₂O₃ und Bi₂O₄; Bismuthydroxid Bi(OH)₃; Bismutchlorid BiCl₃; Bismutbromid BiBr₃, Bismutiodid BiI₃; neutralem Bismutsulfat Bi₂(SO₄)₃; neutralem Bismutnitrat Bi(NO₃)₃ · 5H₂O; Bismutoxidnitrat BiO(NO₃); Bismutoxidcarbonat (BiO)₂CO₃ · 0,5H₂O; Bismutacetat Bi(C₂H₃O₂)₃; Bismutoxidsalicylat C₆H₄CO₂(BiO)OH.

12. Verfahren nach einem der Ansprüche 6 bis 11, dadurch gekennzeichnet, daß die Menge des Aktivators so gewählt ist, das sie in das Medium einbringt: einerseits mindestens 0,1 Gew.-% des Aktivatormetalls im Verhältnis zum Gewicht des beteiligten Metalls M₁ und andererseits 10 bis 900 Gew.-ppm des Metalls M₁ im Verhältnis zur phenolischen Verbindung der Formel (II).

13. Verfahren nach einem der Ansprüche 6 bis 12, dadurch gekennzeichnet, daß die Oxidationsreaktion in einem Temperaturbereich von 30 °C bis 200 °C, vorzugsweise zwischen 40 °C und 160 °C, stattfindet.

14. Verfahren nach einem der Ansprüche 6 bis 13, dadurch gekennzeichnet, daß man bei einem Druck zwischen 1 und 20 bar arbeitet.

15. Verfahren nach einem der Ansprüche 1 bis 14, dadurch gekennzeichnet, daß die Oxidation in einem wäßrigen Medium stattfindet, das in Lösung ein basisches Mittel, vorzugsweise ein Natrium- oder Kaliumhydroxid, einer solchen Menge enthält, daß sie 0,5 bis 10 mol der mineralischen Base pro Mol der phenolischen Verbindung der Formel (II) beträgt.

16. Verfahren nach Anspruch 13 oder 15, dadurch gekennzeichnet, daß im Fall eines Platinkatalysators die Temperatur zwischen 100 °C und 160 °C gewählt ist; die Menge der zu verwendenden Base zwischen 1 und 3 mol pro Mol der phenolischen Verbindungen der Formel (II) liegt.

17. Verfahren nach Anspruch 13 oder 15, dadurch gekennzeichnet, daß im Fall eines Palladiumkatalysators die Temperatur zwischen 30 °C und 200 °C, vorzugsweise zwischen 30 °C und 100 °C, gewählt ist; die Menge der zu verwendenden Base zwischen 2 und 4 mol pro Mol der phenolischen Verbindungen der Formel (II) liegt.

18. Verfahren nach einem der Ansprüche 1 bis 17, dadurch gekennzeichnet, daß man die Decarboxylierung eines teilweise oder völlig in Salzform vorliegenden 3-Carboxy-4-hydroxybenzaldehyds durchführt.

19. Verfahren nach Anspruch 18, dadurch gekennzeichnet, daß das 3-Carboxy-4-hydroxybenzaldehyd der allgemeinen Formel (III) entspricht: 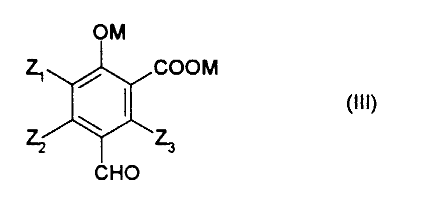 wobei in der genannten Formel (III):
- M ein Wasserstoffatom und/oder ein metallisches Kation der Gruppe (Ia) oder (IIa) oder ein Arnmonium-Kation bedeutet,
- Z₁, Z₂ und Z₃ die in den Ansprüchen 2 bis 4 genannten Bedeutungen haben.

20. Verfahren nach Anspruch 18 oder 19, dadurch gekennzeichnet, daß man die Decarboxylierung der genannten Säure durch Addition einer Protonensäure mineralischen Ursprungs, vorzugsweise Salzsäure oder Schwefelsäure, bis zum Erreichen eines pH-Wertes kleiner oder gleich 3 durchführt.

21. Verfahren nach einem der Ansprüche 18 bis 20, dadurch gekennzeichnet, daß man das Reaktionsgemisch auf eine Temperatur zwischen 120 °C und 350 °C, vorzugsweise zwischen 150 °C und 220 °C, erwärmt und nach dem Abkühlen das 4-Hydroxybenzaldehyd abtrennt, das vorzugsweise der Formel (IV) entspricht: 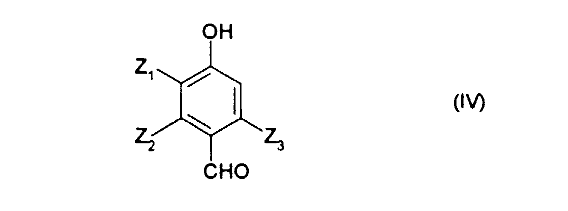 wobei in der Formel (IV):
- Z₁, Z₂ und Z₃ die in den Ansprüchen 2 bis 4 genannten Bedeutungen haben.

22. Verfahren nach einem der Ansprüche 1 bis 21, dadurch gekennzeichnet, daß die ursprünglichen Substrate den Formeln (IIa), (IIb), (IIc) und (IId) entsprechen: 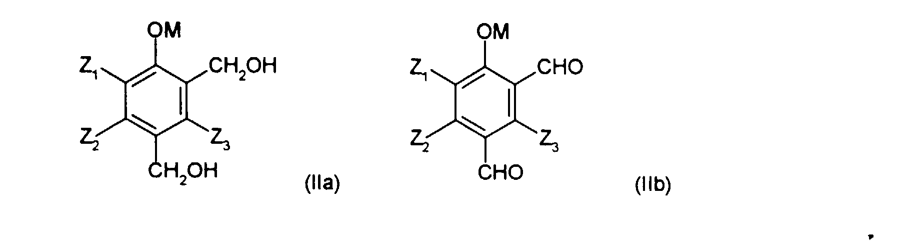 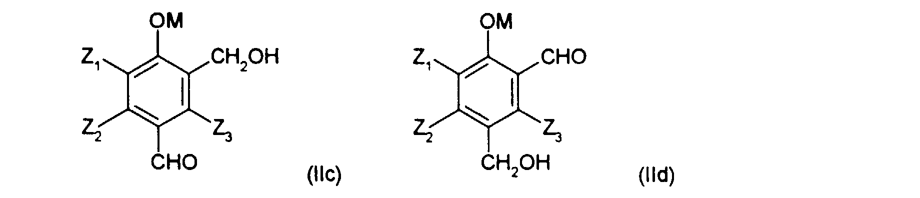 wobei in den genannten Formeln (IIa) bis (IId):
- M ein Wasserstoffatom und/oder ein Metallkation der Gruppe (Ia) oder (IIa) oder ein Ammoniumkation bedeutet,
- Z₁, Z₂ und Z₃ die in den Ansprüchen 2 bis 4 genannten Bedeutungen haben.

23. Verfahren nach Anspruch 22, dadurch gekennzeichnet, daß die Verbindung der Formel (IIb) durch Oxidation von Poly(hydroxymethyl)phenolen der Formel (IIa) mit molekularem Sauerstoff oder einem diesen enthaltenden Gas in alkalischer flüssiger Phase in Gegenwart eines Katalysators auf Grundlage eines Metalls der Gruppe 8 des Periodensystems, vorzugsweise Platin und Palladium, hergestellt wird, der gegebenenfalls als Aktivator Metalle wie Cadmium, Cerium, Bismut, Blei, Silber, Tellur oder Zinn enthält.

24. Verfahren nach Anspruch 22 oder 23, dadurch gekennzeichnet, daß die Poly(hydroxymethyl)phenole der Formel (IIa) durch ein Verfahren mit den folgenden beiden Schritten erhalten werden:
- Hydroxymethylierung eines in ortho- und para-Stellung zur Hydroxylgruppe nicht substituierten Phenols der allgemeinen Formel (I) 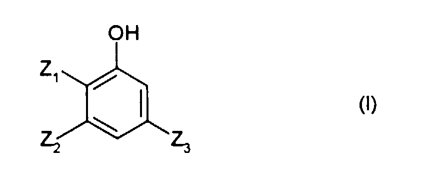 zu einem Poly(hydroxymethyl)phenol, das mindestens eine Hydroxymethylgruppe in ortho- und para-Stellung zur Hydroxylgruppe enthält, der allgemeinen Formel (IIa): 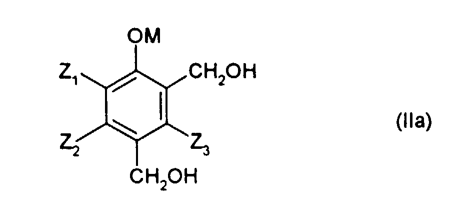 mit Hilfe von Formaldehyd oder einem Formaldehydbildner in wäßriger Phase in Gegenwart einer alkalischen oder erdalkalischen Base;
- Oxidation der aus dem ersten Schritt stammenden Poly(hydroxymethyl)phenole der Formel (IIa) in alkalischer wäßriger Phase mit molekularem Sauerstoff oder einem diesen enthaltenden Gas in Gegenwart eines Katalysators auf Basis eines Metalls der Gruppe 8 des Periodensystems, der gegebenenfalls ein Metall enthält wie die, die als Aktivator verwendet wurden, ohne intermediäre Abtrennung der Poly(hydroxymethyl)phenole.

25. Verfahren nach Anspruch 24, dadurch gekennzeichnet, daß das Phenol der Formel (1) Phenol, Pyrocatechin, Guajakol, 2-Ethoxyphenol, 3-Methoxyphenol, 3-Ethoxyphenol, 3-Isopropoxyphenol, 3-t-Butyloxyphenol, m-Kresol, o-Kresol ist.

26. Verfahren nach Anspruch 24, dadurch gekennzeichnet, daß die Temperatur der Oxidationsreaktion der Verbindungen der Formel (IIa) zwischen 10 °C und 100 °C, vorzugsweise zwischen 20 °C und 60 °C, liegt.

27. Verfahren zur Herstellung von Vanillin nach einem der Ansprüche 1 bis 26, dadurch gekennzeichnet, daß man 4,6-Diformylguajakol oder 4,6-Di(hydroxymethyl)guajakol oder 4-Formyl-6-hydroxymethylguajakol oder 4-Hydroxymethyl-6-formylguajakol zu 5-Carboxy-vanillin oxidiert, anschließend die in 5-Stellung liegende Carboxylgruppe abspaltet und so Vanillin erhält.

28. Verfahren zur Herstellung von Ethylvanillin nach einem der Ansprüche 1 bis 26, dadurch gekennzeichnet, daß man 4,6-Diformyl-2-ethoxyphenol oder 4,6-Di(hydroxymethyl)-2-ethoxyphenol oder 4-Formyl-6-hydroxymethyl-2-ethoxyphenol oder 4-Hydroxymethyl-6-formyl-2-ethoxyphenol zu 5-Carboxyethylvanillin oxidiert, anschließend die in 5-Stellung liegende Carboxylgruppe abspaltet und so Ethylvanillin erhält.

## Claims

1. A process for the preparation of a 3-carboxy-4-hydroxybenzaldehyde and derivatives thereof characterized in that the group in the 2 position in a phenolic compound carrying formyl and/or hydroxymethyl groups in the 2 and 4 positions is selectively oxidised to a carboxy group, and optionally a hydroxymethyl group in the 4 position is selectively oxidised to a formyl group, in the presence of a base and a catalyst based on a metal M₁ selected from metals from groups 1b and 8.

2. A process according to claim 1, characterized in that the phenolic compound has general formula (II): 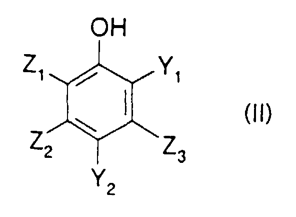 where:
• Y₁ and Y₂, which may be identical or different, represent one of the following groups:
• a -CHO group;
• a -CH₂OH group;
• Z₁, Z₂ and Z₃, which may be identical or different, represent a hydrogen atom, an alkyl, alkenyl, alkoxy, hydroxyalkyl, alkoxyalkyl, cycloalkyl or aryl radical, a hydroxy group, a nitro group, a halogen atom, or a trifluoromethyl group.

3. A process according to claim 1 or claim 2, characterized in that the phenolic compound has formula (II) where Z₁, Z₂, and Z₃, which may be identical or different, represent one of the following atoms or groups:
• a hydrogen atom;
• a linear or branched alkyl radical containing 1 to 12 carbon atoms, preferably 1 to 4 carbon atoms, such as methyl, ethyl, propyl, isopropyl, butyl, isobutyl, sec-butyl or tert-butyl;
• a linear or branched alkenyl radical containing 2 to 12 carbon atoms, preferably 2 to 4 carbon atoms, such as vinyl or allyl;
• a linear or branched alkoxy radical containing 1 to 12 carbon atoms, preferably 1 to 4 carbon atoms, such as a methoxy, ethoxy, propoxy, isopropoxy, butoxy, isobutoxy, sec-butoxy or tert-butoxy radical;
• a phenyl radical;
• a halogen atom, preferably a fluorine, chlorine or bromine atom.

4. A process according to any one of claims 1 to 3, characterized in that the phenolic compound has formula (II) where Z₁ represents a linear or branched alkoxy radical containing 1 to 6 carbon atoms, preferably 1 to 4 carbon atoms; Z₂ and Z₃ represent a hydrogen atom; and Y₁ and Y₂ are identical and represent a formyl group or a hydroxymethyl group.

5. A process according to any one of claims 1 to 4, characterized in that the phenolic compound with formula (II) is:
• 2,4-diformylphenol;
• 1,2-dihydroxy-3,5-diformylbenzene;
• 1 -hydroxy-2-methoxy-4,6-diformylbenzene [4,6-diformylguaiacol];
• 1-hydroxy-2-ethoxy-4,6-diformylbenzene [4,6-diformylguetol];
• 4,6-di(hydroxymethyl)guaiacol;
• 4,6-di(hydroxymethyl)guetol.

6. A process according to any one of claims 1 to 5, characterized in that the phenolic compound with formula (II) is oxidised in the liquid phase using molecular oxygen or a gas containing molecular oxygen, in an aqueous medium comprising a basic agent, in the presence of a catalyst based on a metal M₁ selected from metals from group 1b and 8 of the periodic classification of the elements, which catalyst may contain, as an activator, metals such as cadmium, cerium, bismuth, lead, silver, tellurium or tin.

7. A process according to claim 6, characterized in that the catalyst is based on copper, nickel, ruthenium, rhodium, palladium, osmium, iridium, platinum and mixtures thereof: said catalyst preferably being based on platinum and/or palladium.

8. A process according to claim 6 or claim 7, characterized in that the platinum and/or palladium catalyst is provided in the form of platinum black, palladium black, platinum oxide, palladium oxide or the noble metal itself deposited on different supports such as carbon black, calcium carbonate, aluminas and activated silicas or equivalent materials, preferably carbon black.

9. A process according to any one of claims 6 to 8, characterized in that the quantity of catalyst used, expressed as the weight of metal M₁ with respect to that of the phenolic compound with formula (II), can vary from 0.01% to 10%, preferably 0.04% to 2%.

10. A process according to any one of claims 6 to 9, characterized in that the activator is an organic or inorganic bismuth derivative selected from the group formed by: bismuth oxides; bismuth hydroxides; bismuth or bismuthyl salts of inorganic hydracids, preferably the chloride, bromide, iodide, sulphide, selenide or telluride; bismuth or bismuthyl salts of inorganic oxyacids, preferably the sulphite, sulphate, nitrite, nitrate, phosphite, phosphate, pyrophosphate, carbonate, perchlorate, antimonate, arsenate, selenite, or selenate; bismuth or bismuthyl salts of aliphatic or aromatic organic acids, preferably the acetate, propionate, salicylate, benzoate, oxalate, tartrate, lactate, or citrate; and bismuth or bismuthyl phenates, preferably the gallate or pyrogallate.

11. A process according to claim 10, characterized in that the bismuth derivative is selected from the group formed by: bismuth oxides Bi₂O₃ and Bi₂O₄; bismuth hydroxide Bi(OH)₃; bismuth chloride BiCl₃; bismuth bromide BiBr₃; bismuth iodide BiI₃; neutral bismuth sulphate Bi₂(SO₄)₃; neutral bismuth nitrate Bi(NO₃)₃,5H₂O; bismuthyl nitrate BiO(NO₃); bismuthyl carbonate (BiO)₂CO₃,0.5H₂O; bismuth acetate Bi(C₂H₃O₂)₃ and bismuth salicylate C₆H₄CO₂(BiO)OH.

12. A process according to any one of claims 6 to 11, characterized in that the quantity of activator is selected so that the medium contains: at least 0.1% by weight of metal activator with respect to the weight of metal M₁ used, and 10 to 900 ppm by weight of metal M₁ with respect to the phenolic compound with formula (II).

13. A process according to any one of claims 6 to 12, characterized in that the oxidation reaction is carried out within a temperature range of 30°C to 200°C, preferably between 40°C and 160 °C.

14. A process according to any one of claims 6 to 13, characterized in that the reaction is carried out under a pressure of 1 to 20 bar.

15. A process according to any one of claims 1 to 14, characterized in that the oxidation is carried out in an aqueous medium containing, in solution, a basic agent, preferably sodium or potassium hydroxide, in a quantity such that it represents 0.5 to 10 moles of inorganic base per mole of phenolic compound with formula (II).

16. A process according to any one of claims 13 to 15, characterized in that in the case of a platinum catalyst, the temperature is between 100°C and 160°C; and the quantity of base to be used is in the range 1 to 3 moles per mole of phenolic compounds with formula (II).

17. A process according to any one of claims 13 to 15, characterized in that in the case of a palladium catalyst, the temperature is between 30°C and 200°C, preferably between 30°C and 100°C; and the quantity of base to be used is in the range 2 to 4 moles per mole of phenolic compounds with formula (II).

18. A process according to any one of claims 1 to 17, characterized in that a 3-carboxy-4-hydroxybenzaldehyde is decarboxylated and may be partially or completely in the form of its salt.

19. A process according to claim 18, characterized in that the 3-carboxy-4-hydroxybenzaldehyde has the following general formula (III): 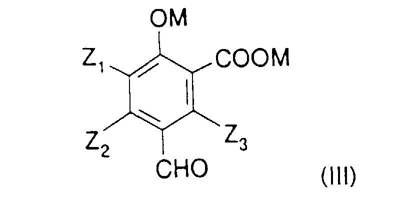 where:
• M represents a hydrogen atom and/or a metal cation from group (Ia) or (IIa) of the periodic classification, or an ammonium cation;
• Z₁, Z₂ and Z₃ have the meanings given in claims 2 to 4.

20. A process according to claim 18 or claim 19, characterized in that the acid is decarboxylated by addition of a protonic acid of inorganic origin, preferably hydrochloric acid or sulphuric acid or an organic acid, until a pH of no more than 3 is attained.

21. A process according to any one of claims 18 to 20, characterized in that the reaction medium is heated to a temperature between 120°C and 350°C, preferably 150°C to 220°C and after cooling, the 4-hydroxybenzaldehyde is separated which preferably has the following formula (IV): 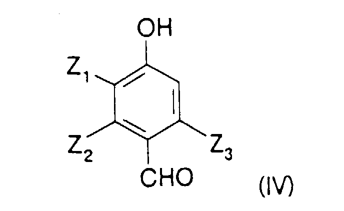 where:
• Z₁, Z₂ and Z₃ have the meanings given in claims 2 to 4.

22. A process according to any one of claims 1 to 21, characterized in that the starting substrates have formulae (IIa), (IIb), (IIc) and (IId): 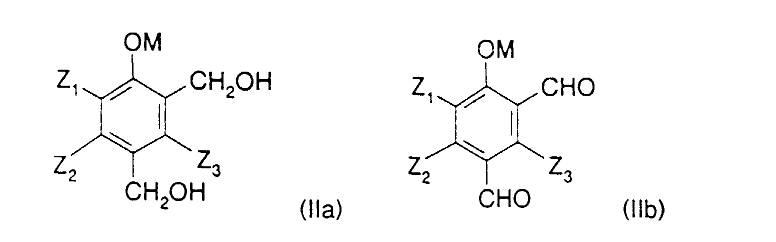 where: 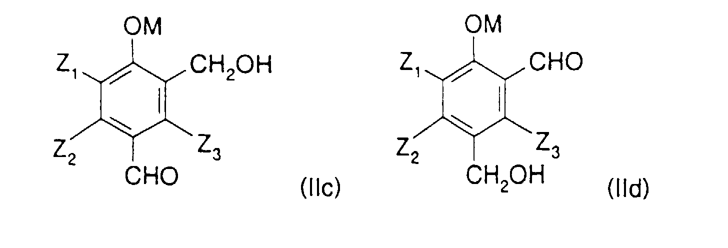
• M represents a hydrogen atom and/or a metal cation from group (Ia) or (IIa) of the periodic classification, or an ammonium cation;
• Z₁, Z₂ and Z₃ have the meanings given in claims 2 to 4.

23. A process according to claim 22, characterized in that the compound with formula (IIb) is prepared by oxidation of poly(hydroxymethyl)phenols with formula (IIa) by molecular oxygen or a gas containing molecular oxygen, in an aqueous alkaline phase in the presence of a catalyst based on a metal from group 8 of the periodic classification, preferably platinum or palladium, which may contain, as an activator, metals such as cadmium, cerium, bismuth, lead, silver, tellurium or tin.

24. A process according to claim 22 or claim 23, characterized in that the poly(hydroxymethyl)phenols with formula (IIa) are obtained by a two-step process comprising:
• hydroxymethylation at the ortho and para positions to the hydroxy group of an unsubstituted phenol with general formula (I): 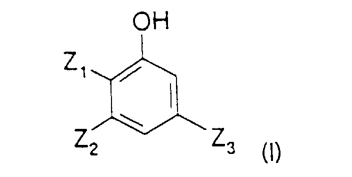 to form a poly(hydroxymethyl)phenol containing at least one hydroxymethyl group in the ortho and para position to the hydroxy group, with general formula (IIa): 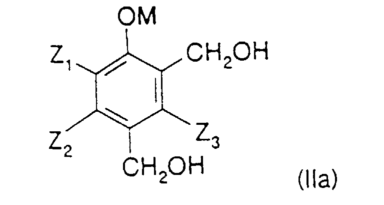 using formaldehyde or a formaldehyde generator in an aqueous phase in the presence of an alkaline or alkaline-earth base;
• oxidation, in the aqueous alkaline phase, of the poly(hydroxymethyl)phenols with formula (IIa) from the first step using molecular oxygen or a gas containing molecular oxygen in the presence of a catalyst based on a metal from group 8 of the periodic classification, optionally containing a metal such as those used as an activator, without intermediate preparation of poly(hydroxymethyl)phenols.

25. A process according to claim 24, characterized in that the phenol with formula (I) is phenol, pyrocatechin, guaiacol, guetol, 3-methoxyphenol, 3-ethoxyphenol, 3-isopropoxyphenol, 3-t-butyloxyphenol, m-cresol or o-cresol.

26. A process according to claim 24, characterized in that the reaction temperature for oxidation of compounds with formula (IIa) is between 10°C and 100°C, preferably between 20°C and 60°C.

27. A process for the preparation of vanillin in accordance with any one of claims 1 to 26, characterized in that 4,6-diformylguaiacol or 4,6-di(hydroxymethyl)guaiacol or 4-formyl-6-hydroxymethylguaiacol or 4-hydroxymethyl-6-formylguaiacol is oxidised to 5-carboxy-vanillin, then the carboxy group in the 5 position is eliminated to produce vanillin.

28. A process for the preparation of ethylvanillin in accordance with any one of claims 1 to 26, characterized in that 4,6-diformylguetol or 4,6-di(hydroxymethyl)guetol or 4-formyl-6-hydroxymethylguetol or 4-hydroxymethyl-6-formylguetol is oxidised to 5-carboxy-ethylvanillin, then the carboxy group in the 5 position is eliminated to produce ethylvanillin.
